# EUROPEAN PATENT APPLICATION

(11) **EP 4 141 105 A1**
(43) Date of publication of application: **01.03.2023**
(21) Application number: 21792497.6
(22) Date of filing: 14.04.2021
(51) Int. Cl.: C12N 5/02, C12N 5/0783

(54) **UNIVERSAL-TYPE EFFICIENT IN-VITRO AMPLIFICATION METHOD FOR MULTIPLE TIMES OF CLINICAL FEEDBACK OF ALLOGENIC DNT CELLS**

(30) Priority: 20.04.2020 CN 202010313732
(71) Applicant: Ruichuang Biotech. Co., Ltd., Shaoxing, Zhejiang 312030 (CN)
(72) Inventor: YANG, Liming, Shaoxing, Zhejiang 312030 (CN); XIANG, Zhiqiang, Shaoxing, Zhejiang 312030 (CN); SUN, Qinghua, Shaoxing, Zhejiang 312030 (CN)
(74) Representative: Cabinet Laurent & Charras
(86) International application number: PCT/CN2021/087312
(87) International publication number: WO 2021/213236

(57) **Abstract**

The present invention relates to a universal-type efficient in-vitro amplification method for multiple times of clinical feedback of allogenic DNT cells. In the method of the present invention, the amount of a T cell activator is phased down in a continuous culture process without the need for additional interleukin-4 or AB serum.

## Description

### Technical field

The present invention belongs to the field of biotechnology and specifically relates to a method for universal-type efficient in vitro expansion for the clinical multiple transfusion of allogeneic DNT cells.

### Background

Immune cell therapy is an emerging tumor treatment model with significant efficacy and is a novel approach to immunotherapy against cancer. It is the use of biotechnology and biological agents to culture and amplify immune cells collected from a patient or healthy donor in vitro and then transfuse them back into the patient to stimulate and enhance the body's own immune function for the purpose of treating tumors.

Immune cell therapy is an important treatment for many serious human diseases, including cancer, autoimmune diseases, organ transplant rejection, severe allergic diseases and even severe viral infections have been successfully treated with immune cells.

Currently, immune cell therapy mostly involves the collection of autologous immune cells for in vitro culture and expansion, followed by transfusion. If multiple transfusions are required, immune cells from human peripheral blood have to be collected several times for culture and expansion to meet the clinical demand for transfusion. Due to the severity of the disease, most patients requiring treatment are often unable to provide sufficient quantities of immune cells for culture, and the patient's condition and various medication treatments make it very difficult or even impossible to culture and expand immune cells in vitro.

Therefore, there is a need in the field for a universal method for the in vitro expansion of immune cells from healthy donors that can be used for multiple transfusions to facilitate clinical treatment and avoid the clinical inconvenience of multiple collection-multiple transfusions, while eliminating the risk of tumor cells being present due to the poor quality of peripheral blood from cancer patients.

### Summary of the invention

The purpose of the present invention is to provide a novel method for universal-type efficient in vitro expansion for the clinical multiple transfusion of allogeneic DNT cells.

In a first aspect of the present invention, it provides an in vitro expansion method of a universal DNT cell, comprising the steps of:
(i) providing a starting sample I of peripheral blood obtained from a donor.
(ii) removing CD4⁺ and CD8⁺ T cells from the starting sample, thereby obtaining a sample II.
(iii) culturing the sample II in a culture system containing a medium suitable for the growth of a DNT cell, thereby obtaining a sample III; wherein the culture system comprises immobilized T-cell mitogen;
(iv) culturing the sample III in a culture system containing a medium suitable for DNT cell growth, thereby obtaining a sample IV; wherein the medium contains a soluble T-cell mitogen at a concentration of 30-80 ng/mL (preferably 40-60 ng/mL, more preferably 50 ng/mL).
(v) culturing the sample IV in a culture system containing a medium suitable for DNT cell growth, wherein the medium contains soluble T-cell mitogen at a concentration of 15-40 ng/mL (preferably 20-30 ng/mL, more preferably 25 ng/mL, more preferably soluble T-cell mitogen in an amount 1/2 of the added amount of the T-cell mitogen as described in sub-step (va), thereby obtaining a desired amount of universal DNT cell, as a sample V; and
(vi) collecting the sample V in a system containing a solution suitable for the preservation of the DNT cell; wherein the solution contains a human albumin, thereby obtaining the universal DNT cell for clinical use, as a DNT cell preparation;
wherein the culture systems of steps (iii) to (v) all contain 200-1000 IU/mL (preferably 300-700 IU/mL, more preferably 500 IU/mL) of recombinant human interleukin 2, and all do not contain recombinant human interleukin 4, and all do not contain AB serum.

In another preferred embodiment, the donor in the step (i) is a healthy donor.

In another preferred embodiment, the amount of the sample I is 10-300 ml, preferably 20-200 ml.

In another preferred embodiment, the sample II is a solution of recovered cell after cryopreservation.

In another preferred embodiment, the step (ii) comprises: removing the CD4⁺ and CD8⁺ T cell from the starting sample to obtain a sample IIa (the enriched DNT cell); after centrifugation and collection of the sample IIa (the enriched DNT cell) resuspension in a freezing solution, programmed to cool down and stored in liquid nitrogen, as a sample IIb (enrichment of the frozen DNT cell); after freezing, after freezing of the sample IIb, sample IIb is thawed at 37°C, washed once with culture medium and resuspended in culture medium, which is a sample IIc (enrichment of frozen and resuscitated DNT cells).

In a further preferred embodiment, in the culture system of the steps (iii) to (v), the culture condition is 37°C, 5% CO₂.

In a further preferred embodiment, in the culture medium of the steps (iii) to (v), further comprising a cytokine selected from the group consisting of: IL-7, IL-12, IL-15, and a combination thereof.

In a further preferred embodiment, before the step (iii), further comprising a step (iia): washing the sample II with 0.9% saline.

In another preferred embodiment, the T cell mitogen is selected from the group consisting of: an antibody binding CD3, a lectin, a compound capable of stimulating the expansion of DNT cells, and a combination thereof.

In a further preferred embodiment, the lectin is selected from the group consisting of: plant lectin concanavalin A (ConA), plant lectin (PHA), soybean lectin (SBA), and a combination thereof.

In a further preferred embodiment, the compound capable of stimulating the expansion of DNT cells is selected from the group consisting of: IPP, pamidronic acid (Pamidronate), zoledronic acid (Zoledronate), and a combination thereof.

In a further preferred example, the T cell mitogen is an antibody that binds CD3.

In a further preferred embodiment, the culture system in the step (iii) is located in a culture flask, preferably a T25 culture flask.

In a further preferred embodiment, the immobilized T cell mitogen is a T-cell mitogen encapsulated on a culture flask or microtiter plate.

In another preferred embodiment, in the step (iii), the initial concentration of DNT cells to be expanded in the culture system is from 1 × 10⁶ to 4 × 10⁶ cells/mL.

In another preferred embodiment, the incubation time for the step (iii) is 24-72 hours, preferably 36-60 hours, more preferably 48 hours.

In a further preferred embodiment, in step (iii), the medium is supplemented with 15-25% (preferably 18-22%, more preferably 20%) of the plasma from the donor.

In another preferred example, in the step (iii), divided into sub-steps (iiia), (iiib) and (iiic), the sub-steps (iiia), (iiib) and (iiic) being three amplifications of the DNT cells in sample III.

In another preferred example, in sub-step (iiia), the initial concentration of DNT cells to be expanded in the culture system is 1 × 10⁶ to 4 × 10⁶ cells/mL.

In another preferred embodiment, in sub-steps (iiia) and (iiib), the initial concentration of DNT cells to be expanded in the culture system is each independently 0.5 × 10⁶ to 1 × 10⁶ cells/mL.

In a further preferred example, the incubation time for the sub-steps (iiia), (iiib) and (iiic) is each independently 24-72 hours, preferably 36-60 hours, more preferably 48 hours.

In another preferred example, the incubation time for the step (iii) is 96-192 hours, preferably 120-168 hours, more preferably 144 hours.

In another preferred embodiment, in step (iv), comprising 2-5 (preferably 3-4, more preferably 3) expansions of DNT cells, in each of which the initial concentration of DNT cells to be expanded is each independently 1 × 10⁶ to 3 × 10⁶ cells/mL, and the incubation time for each expansion is 24-72 hours, preferably 36-60 hours, more preferably 48 hours.

In another preferred embodiment, in step (iv), further comprising the detection of DNT cell phenotype and viability.

In a further preferred embodiment, the incubation time for the step (iv) is 72-168 hours, preferably 96-144 hours, more preferably 120 hours.

In a further preferred embodiment, in step (v), comprising 2-4 (preferably 2) expansions of DNT cells, in each expansion, the initial concentration of DNT cells to be expanded is each independently 1 × 10⁶ to 3 × 10⁶ cells/mL and the culture time for each expansion is 24-72 hours, preferably 36 hours.

In another preferred embodiment, in step (v), further comprising the detection of DNT cell phenotype and viability.

In a further preferred embodiment, the incubation time for the step (v) is 48-96 hours, preferably 72 hours.

In a further preferred embodiment, in step (vi), comprising the steps of:
(via) collecting the DNT cells in the sample V by centrifugation.
(vib) washing the DNT cells with saline (or "solvent") containing 2.5% human albumin; and
(vic) adjusting the DNT cells to a concentration of 1 × 10⁸ cells/mL with saline (or "solvent ") containing 2.5% human albumin.

In a second aspect of the present invention, it provides a use of an effective amount of the DNT cell obtained by the method as described in the first aspect of the present invention for the preparation of a pharmaceutical composition or a preparation, the pharmaceutical composition or the preparation being used for:
(a) the prevention and/or treatment a tumor.
(b) the prevention and/or treatment of an infectious disease.
(c) the prevention and/or treatment of an autoimmune disease.
(d) the prevention and/or treatment of an allergic reaction.
(e) the prevention and/or treatment of a graft versus host disease; and/or
(f) the regulation of an immune response.

In a further preferred embodiment, the tumor is a tumor that is allogeneic to the DNT cell.

In a further preferred embodiment, the tumor is selected from the group consisting of: hematologic neoplasms, solid tumor, and a combination thereof.

In a further preferred embodiment, the hematologic neoplasms is selected from the group consisting of: lymphomas (Hodgkins and non-Hodgkins), acute myelocytic leukemia (AML), multiple myeloma (MM), chronic lymphocytic leukemia (CLL), acute lymphoid leukemia (ALL), diffuse large B-cell lymphoma (DLBCL), chronic myelogenous leukemia (CML), Chronic myelomonocytic leukaemia (CMML), myelodysplastic syndrome (MDS), and a combination thereof.

In another preferred embodiment, the solid tumor is selected from the group consisting of: gastric cancer, peritoneal metastasis from gastric cancer, liver cancer, leukemia, kidney tumor, lung cancer, carcinoma of small intestine, melanoma, bone cancer, prostate cancer, colorectal cancer, breast cancer, colorectal cancer, cervical cancer, ovarian cancer, lymphoma, nasopharynx cancer, adrenal tumor, bladder tumor, non-small cell lung cancer (NSCLC), glioma, head and neck cancer, pancreatic cancer, and a combination thereof.

In another preferred embodiment, the autoimmune disease comprises: diabetes mellitus, arthritis, multiple sclerosis, lupus erythematosus, inflammatory bowel disease, dermatitis, meningitis, thrombotic thrombocytopenic purpura, Sjögren's syndrome, encephalitis, ocular uveitis, leukocyte adhesion deficiency, rheumatic fever, Reiter's syndrome, progressive systemic sclerosis, primary biliary cirrhosis, necrotizing vasculitis, myasthenia gravis, polymyositis, sarcoidosis, granulomatosis, vasculitis, pernicious anemia, CNS inflammatory disease, antigen-antibody complex-mediated disease, autoimmune hemolytic anemia, struma lymphomatosa, exophthalmic goiter, recurrent spontaneous abortions, Raynaud's syndrome, glomerulonephritis, dermatomyositis, chronic active hepatitis, celiac disease, tissue-specific autoimmunity, degenerative autoimmune delayed hypersensitivity reaction, autoimmune complications of AIDS, atrophic gastritis, ankylosing spondylitis, Addison's disease, and a combination thereof.

In another preferred example, the allergic reaction includes: hay fever, asthma, atopic eczema, allergic reaction to rhus diversiloba and ivy, house dust mites, bee pollen, nuts, crustaceans, penicillin, and a combination thereof.

In a third aspect of the present invention, it provides a use of a DNT cell obtained by the method as described in the first aspect of the present invention for:
(a) the prevention and/or treatment a tumor.
(b) the prevention and/or treatment of an infectious disease.
(c) the prevention and/or treatment of an autoimmune disease.
(d) the prevention and/or treatment of an allergic reaction.
(e) the prevention and/or treatment of a graft versus host disease; and/or
(f) the regulation of an immune response.

It should be understood that, within the scope of the present invention, each technical feature of the present invention described above and in the following (as examples) may be combined with each other to form a new or preferred technical solution, which is not listed here due to space limitations.

### Description of Figure

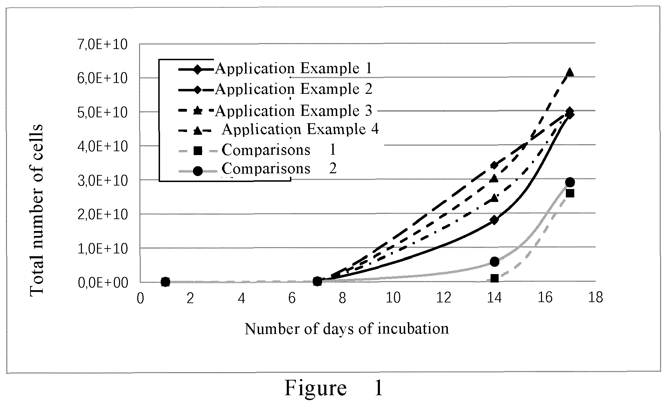

Figure 1 shows the cell growth curves for Application Examples 1-4 versus Comparisons 1 and 2; specifically, the cells from the Application Examples and the Comparisons are sampled on day 1, day 7, day 14 and day 17 of culture, respectively for the detection of total number of cells and the results of changes in the total number of cells are compared.

### Detailed Description

After extensive and in-depth research and extensive process optimization experiments, the present inventors have developed for the first time a practical and efficient in vitro expansion method for obtaining donor peripheral blood for clinical multiple transfusion of allogeneic DNT cells at one time. Specifically, the present invention optimizes the culture formulation and culture process of DNT cells. Compared to existing in vitro expansion methods of DNT cells, the method of the invention does not require additional addition of IL-4 and decreases the amount of T cell activator (anti-human CD3 antibody, herein) in stages over the course of successive cultures, high purity universal DNT cells can be obtained, greatly reducing the amount of impurities in the final product.

The results show that the method of the present invention can be used for the efficient in vitro expansion of freshly collected and enriched DNT cells from human peripheral blood, and also for the efficient in vitro expansion of DNT cells after subsequent resuscitation of the aforementioned DNT cells that were first frozen. The process of the invention results in a large number of DNT cells and a high purity (>85%) of CD3⁺CD4⁻CD8⁻ T cells, a characteristic surface marker of DNT cells is obtained. Since the tumor-killing activity of DNT cells is not dependent on the T-cell receptor, DNT cells prepared using the same donor can be provided to different patients for clinical treatment.

On this basis, the present invention is completed.

### Terms

In order that the present disclosure may be more readily understood, certain terms are first defined. As used in this application, each of the following terms shall have the meaning given below, unless otherwise expressly provided herein. Other definitions are set forth throughout the application.

As used herein, the term "about" may refer to a value or composition within an acceptable margin of error for a particular value or composition as determined by one of ordinary skill in the art, which will depend in part on how the value or composition is measured or determined.

As used herein, the terms "giving", "administering" are used interchangeably and refer to the physical introduction of the product of the present invention into a subject using any one of a variety of methods and delivery systems known to those of skill in the art, including intravenous, intramuscular, subcutaneous, intraperitoneal, spinal or other parenteral routes of administration, for example by injection or infusion.

### In vitro expansion method of the present invention

In the present invention, it provides a method for the in vitro bulk expansion of universal DNT cells, the method comprising the steps of:
(i) providing a starting sample I of peripheral blood obtained from a donor;
(ii) removing CD4⁺ and CD8⁺ T cells from the starting sample, thereby obtaining a sample II;
(iii) culturing the sample II in a culture system containing a medium suitable for the growth of DNT cells, thereby obtaining a sample III; wherein the culture system comprises an immobilized T cell mitogen;
(iv) culturing the sample III in a culture system containing a medium suitable for DNT cell growth, thereby obtaining a sample IV; wherein the medium contains a soluble T cell mitogen at a concentration of 30-80 ng/mL (preferably 40-60 ng/mL, more preferably 50 ng/mL).
(v) culturing the sample IV in a culture system containing a culture medium suitable for the growth of DNT cells, wherein the culture medium contains soluble T-cell mitogen at a concentration of 15-40 ng/mL (preferably 20-30 ng/mL, more preferably 25 ng/mL, more preferably soluble T-cell mitogen in an amount 1/2 of the added amount of the T-cell mitogen as described in sub-step (va), thereby obtaining a desired amount of universal DNT cell, as a sample V; and
(vi) collecting the sample V in a system containing a solution suitable for the preservation of the DNT cell; wherein the solution contains a human albumin, thereby obtaining the universal DNT cell for clinical use, as a DNT cell preparation;
wherein the culture systems of steps (iii) to (v) all contain 200-1000 IU/mL (preferably 300-700 IU/mL, more preferably 500 IU/mL) of recombinant human interleukin 2, and all do not contain recombinant human interleukin 4, and all do not contain AB serum.

As used herein, the terms "double negative T cells" and "DNT cells" are used interchangeably to refer to a subpopulation of T lymphocytes that express CD3 molecules on their cell surface but lack CD4, CD8 molecules and CD16/CD56 molecules. Wherein CD4 is a characteristic surface molecule for helper T cells, CD8 is a characteristic surface molecule for cytotoxic T cells and CD16/CD56 is a characteristic surface molecule for NK cells. The DNT cells express a T cell receptor (TCR), which may be either an αβ or γδ TCR. The DNT cell subpopulation expanded by the method of the invention may comprise a mixture of αβ⁺ and γδ⁺ T cells. In a preferred embodiment, the DNT cells are cells derived from human peripheral blood.

The starting sample may be derived from any biological sample containing double negative T cells or their precursors. Such samples include, but are not limited to, fresh or cryopreserved blood, bone marrow, lymphoid tissue, thymus, liver, spleen, lymph node tissue, tumor tissue, fetal tissue cells and graded or enriched fractions thereof, and may also be derived from induced Pluripotent Stem Cells (iPSC).

In a preferred embodiment, the starting sample is blood, preferably human blood, more preferably human peripheral blood.

The starting sample is substantially free of CD4⁺ and CD8⁺ T cells prior to culturing the starting sample or a fraction thereof. "Substantially" means that the majority of these cells are removed but does not exclude that a small proportion of these cells remain.

These cell types can be removed from the sample using techniques known in the art. Specifically, antibodies may be added to the starting sample, the antibodies binds the CD8⁺ and CD4⁺ cells to be removed but not the double negative T cells. In a preferred embodiment, an antibody specifically binding a marker for CD4 and CD8 is added to the sample.

In another preferred embodiment, magnetic beads that specifically bind CD4 and CD8 are added to the sample.

Once the CD4⁺ and CD8⁺ T cells have been removed from the starting sample, the method of the invention can be continued immediately or the sample can be frozen and stored, either in a cryogenic refrigerator or device (below -80°C) or in liquid nitrogen, for later use. Thus when DNT cells are required, the in vitro expansion of DNT cells can be started from a frozen sample at this time.

In one embodiment of the present invention, it provides a method for obtaining peripheral blood (100-400 ml) from a healthy donor at one time, enriching DNT cells in vitro and immediately freezing them, and recovering this enriched DNT cells for in vitro expansion of the present invention according to a clinical transfusion protocol.

Preferably, step (ii) of the method of the present invention may comprise: removing CD4⁺ and CD8⁺ T cells from the starting sample, thereby obtaining sample IIa (enriched DNT cells); sample IIa may be cultured directly by the in vitro expansion method of the present invention, or may be recovered after freezing in liquid nitrogen and then cultured; sample IIa (enriched DNT cells) is collected by centrifugation, resuspended in lyophilized solution, programmed cooling and storage in liquid nitrogen, this is sample IIb (enriched lyophilized DNT cells); sample IIb was frozen and recovered and cultured according to clinical needs; after thawed at 37°C, washed once with culture medium and resuspended in culture medium, which is a sample IIc (enrichment of frozen and resuscitated (recovered) DNT cells).

Samples in which CD4⁺ and CD8⁺ cells have been substantially removed are cultured in a medium comprising an immobilized T-cell mitogen and a reagent capable of stimulating DNT cell growth.

The immobilized T-cell mitogen can be any reagent capable of stimulating double negative T cells, including but not limited to antibodies that bind CD3 or T-cell receptors and lectins including plant lectin concanavalin A (ConA) and plant lectin (PHA) or any compound capable of stimulating DNT cell expansion such as but not limited to IPP, pamidronic acid (Pamidronate), zoledronic acid (Zoledronate). Preferably, the T cell mitogen is an antibody to CD3 such as OKT3.

T-cell mitogen can be immobilized using techniques well known in the art. Preferably, the T-cell mitogen is encapsulated into a solid phase support, including but not limited to a microtiter plate, culture dish, culture bag, or culture flask. Preferably, the T-cell mitogen is an immobilized anti-CD3 antibody, more preferably immobilized on a microtiter plate.

The reagent capable of stimulating DNT cell growth may be any suitable reagent, preferably a cytokine, such as interleukin. Preferably, the cytokines include interleukin-2 (IL-2), interleukin-7 (IL-7), interleukin-12 (IL-12), interleukin-15 (IL-15), or a mixture of two or more of these.

Particularly noteworthy, in the present invention, the medium used for expansion culture does not contain IL-4 and does not contain AB serum.

The concentration of the reagent should be suitable to promote the expansion of double negative cells. Preferably, the cytokine is administered in a range from about 200 IU/mL to 1000 IU/mL. In a particularly preferred embodiment, the concentration of IL-2 in the medium is 500 IU/mL.

The medium may be any medium suitable for T cell culture, including but not limited to AIM-V medium, RPMI medium, X-VIVOI0 and X-VIVOI5, and any other commercially available animal serum-free medium. The medium preferably contains other suitable reagents including antibiotics.

In one embodiment, the cells may be co-cultured with DC cells or stimulated with antigens and cytokines when cultured with reagents capable of stimulating DNT cell growth and immobilized T cell mitogen. In the preparation of anti-tumor DNT cells, inactivated tumor cells or tumor-specific or tumor-associated antigens, peptides or neoantigen may be used.

The cells are preferably cultured in any one of steps (ii)-(v) for a period of time ranging from about 2-8 days. Preferably, each step is carried out for about 3-7 days, more preferably 4-6 days.

The purity of DNT cells prepared by this method can be confirmed using techniques known in the art such as flow cytometry or other live cell phenotype identification techniques.

### Uses of the DNT cells prepared by the present invention

The present invention also comprises the use of double negative T cells obtained by the method of the present invention in any and all applications. The allogeneic T cells prepared by the method of the present invention can be used for a wide range of tumor treatments, can be prepared on a large scale, are of stable and controlled quality and can be administered to any applicable patient in a timely manner.

In embodiments of the present invention, the double negative T cells expanded by the method of the present invention have a strong anti-tumor effect. Thus, in one embodiment, the present invention provides a method of treating a tumor comprising administering an effective amount of universal DNT cells obtained by the method of the present invention to an animal in need thereof. The present invention also includes the use of an effective amount of universal DNT cells obtained by the method of the present invention for the treatment of tumors. The present invention also includes the use of an effective amount of universal DNT cells obtained by the method of the present invention in the preparation of drugs for the treatment of tumors.

The term "effective amount" as used herein refers to a dose that is effective in achieving the desired result, for example the treatment of cancer, at the dose and in the time required.

The term "animal" as used herein includes all members of the animal kingdom, including humans. In a preferred embodiment, said animal is a human.

The term "treatment" includes, but is not limited to, the reduction or improvement of one or more symptoms of a disease or condition (e.g. cancer, transplant rejection and graft versus host disease, autoimmune disease, allergic reactions, infections, etc.), reduction in the extent of the disease, stabilization state of the disease, prevention of the spread of disease, delay or slowing of disease progression and improvement or remission of disease state, detectable or non-detectable symptom relief and/or prolonged survival compared to what would have been expected if the treatment had not been received.

In the treatment of tumors or cancers, a treatable tumor is any tumor that can be treated with double negative T cells alone or in combination with other treatments such as surgery, radiotherapy or chemotherapy, including various targeted therapeutic agents, immune checkpoint inhibitors, immune cell enhancers and nanomaterials that enhance infiltration of DNT cells in tumor tissue.

Treatable cancers include tumors that have not been vascularized or have not been substantially vascularized, as well as vascularized tumors. Cancers may include non-solid tumors (such as hematologic neoplasms, for example leukemia and lymphoma) or may include solid tumors. The types of cancer treated with the present invention include, but are not limited to, carcinomas, enblastoma and sarcomas, and certain leukemia or lymphoid malignancies, benign and malignant tumors, and malignant tumors such as sarcomas, carcinomas and melanomas. It also includes adult tumors/cancers and pediatric tumors/cancers.
hematologic neoplasms are cancers of the blood, bone marrow, or lymphoid tissue. Examples of hematologic (or hematogenous) cancers include leukemia, including acute leukemia (such as acute lymphoblastic leukemia, acute myelocytic leukemia, acute myelogenous leukemia and myeloblastic, promyelocytic, granule-monocyte type, monocytic leukemia and erythroleukemia), chronic leukemia (such as chronic myeloid (myelogenous) leukemia, chronic myelogenous leukemia and chronic lymphocytic leukemia), polycythemia vera, lymphoma, Hodgkin's disease, non-Hodgkin's lymphoma (painless and high-grade forms), multiple myeloma, Waldenström's macroglobulinemia, heavy chain disease, myelodysplastic syndrome, hairy cell leukemia and myelodysplasia.

Solid tumors are abnormal masses of tissue that do not usually contain cysts or areas of fluid. Solid tumors can be benign or malignant. The different types of solid tumors are named after the type of cells that form them (such as sarcomas, carcinomas and lymphomas). Examples of solid tumors such as sarcomas and carcinomas include fibrosarcoma, myxosarcoma, liposarcoma mesothelioma, lymphoid malignancies, and pancreatic cancer ovarian cancer.

The invention also includes other therapeutic uses of DNT cells, it is used, for example, in the treatment of infectious diseases and in the regulation of the immune response, for example, in the treatment of autoimmune diseases, allergic reactions, transplant rejection and graft-versus-host disease. In this context, the method of preparing DNT cells may comprise the addition of a suitable infectious substances, archaeocyte of allergic reaction or tissue as antigen.

Thus, in one embodiment, the present invention provides a method of treating an infectious disease comprising administering an effective amount of double negative T cells obtained by the method of the present invention. The present invention also includes the use of an effective amount of double negative T cells obtained by the method of the invention for the treatment of infectious diseases. The present invention also includes the use of an effective amount of the double negative T cells obtained by the method of the present invention in the preparation of a drug for the treatment of an infectious disease.

In a further embodiment, the present invention provides a method of modulating an immune response comprising administering an effective amount of double negative T cells obtained by the method of the present invention. The present invention also includes the use of an effective amount of double negative T cells obtained by the method of the present invention for the regulation of an immune response. The present invention also includes the use of an effective amount of the double negative T cells obtained by the method of the present invention in the preparation of a drug for the regulation of an immune response.

In one embodiment, DNT cells are used in the treatment of autoimmune diseases. Autoimmune diseases that can be treated according to the present invention include, but are not limited to, diabetes, arthritis, multiple sclerosis, lupus erythematosus, inflammatory bowel disease, dermatitis, meningitis, thrombotic thrombocytopenic purpura, Sjögren's syndrome, encephalitis, ocular uveitis, leukocyte adhesion deficiency, rheumatic fever, Reiter's syndrome, progressive systemic sclerosis, primary biliary cirrhosis, necrotizing vasculitis, myasthenia gravis, polymyositis, sarcoidosis, granulomatosis, vasculitis, pernicious anemia, CNS inflammatory disease, antigen-antibody complex-mediated disease, autoimmune hemolytic anemia, struma lymphomatosa, exophthalmic goiter, recurrent spontaneous abortions, Raynaud's syndrome, glomerulonephritis, dermatomyositis, chronic active hepatitis, celiac disease, tissue-specific autoimmunity, degenerative autoimmune delayed hypersensitivity reaction, autoimmune complications of AIDS, atrophic gastritis, ankylosing spondylitis and Addison's disease.

In another embodiment, DNT cells can be used to treat graft-versus-host disease, in which immune cells in the graft are immune attacked against normal tissue of the recipient. This may be the case when the tissue being transplanted contains immune cells, for example when bone marrow or lymphoid tissue from a healthy donor is transplanted for the treatment of leukemia, aplastic anaemia and enzymes or immune deficiencies.

In further embodiments, DNT cells can be used to treat allergic reaction. In a allergic reaction, the immune system attacks an antigen or allergens that are normally non-toxic and harmless. Allergic reaction that can be prevented or treated using the methods of the present invention includes, but is not limited to, hay fever, asthma, atopic eczema, allergic reaction to rhus diversiloba and ivy, house dust mites, bee pollen, nuts, crustaceans, penicillin and many other substances.

The DNT cells prepared by the method of the present invention can be formulated into pharmaceutical compositions that are given to the subject in a biocompatible form suitable for in vivo administration. The term "biocompatible form suitable for in vivo administration" refers to a form of the substance being administered in which the therapeutic effect exceeds any toxic effect. The substance may be administered to living organisms, including humans and animals. The composition may be administered in a suitable manner, preferably by injection, e.g. intravenously, subcutaneously, intramuscularly, etc.

The compositions described herein may be prepared by methods otherwise known for the preparation of pharmaceutically acceptable compositions, said compositions being capable of being given to a subject such that an effective amount of cells is combined with a pharmaceutically acceptable carrier as a mixture. For example Remington's Pharmaceutical Sciences (Remington's Pharmaceutical Sciences, 20th ed., Mack Publishing Company, Easton , Pa., USA 2000) describes suitable carriers. On this basis, the composition comprises, but is not limited to, a solution of the substance in combination with one or more pharmaceutically acceptable carriers or diluents, containing a physiological solution in a buffered solution having a suitable pH and isotonicity.

The effective amount of the composition may vary depending on factors such as the individual's disease state, age, gender and body weight and the ability of the cells to trigger the desired response in the individual. Dosing regimens can be adjusted to provide an optimal therapeutic response. For example, several separate doses may be administered weekly, or the dose may be reduced proportionally as required by the therapeutic situation.

Drugs that can be used in combination with the present invention include other active substances that can be used to treat the disease or condition to be treated. For example, in oncology treatment, other anti-cancer agents may be given in the same composition or in separate compositions.

The pharmaceutical compositions of the invention can be administered in a manner suitable for the disease to be treated (or prevented). The amount and frequency of administration will be determined by such factors as the patient's condition, the type and severity of the patient's disease - although the appropriate dose may be determined by clinical trials.

When "immunologically effective amount", "antitumor effective amount", "tumor-inhibiting effective amount" or "therapeutic amount " is indicated, the precise amount of the composition of the invention to be administered may be determined by clinical trials, taking into account individual differences in the age, weight, tumor size, degree of infection or metastasis and disease of the patient (subject). It may be generally noted that: pharmaceutical compositions comprising the T cells described herein may be administered at doses of 10⁴ to 10⁹ cells/kg body weight, preferably 10⁶ to 10⁸ cells/kg body weight (including all integer values in those ranges). The T cell compositions may also be administered multiple times at these doses. Cells can be administered by using injection techniques well known in immunotherapy (see e.g. Rosenberg et al., New Eng. J. of Med. 319:1676, 1988). The optimal dose and treatment regimen for a specific patient can be obtained by monitoring the patient's disease status, so that therapeutic modifications are readily determined by those skilled in the medical field.

Administration of the subject composition may be carried out in any convenient manner, including by spray method, injection, swallowing, infusion, implantation or transplantation. The compositions described herein may be administered subcutaneously, intradermally, intratumorally, intralymph node, intraspinal, intramuscularly, by intravenous (i.v.) injection or intraperitoneally to the patient. In one embodiment, the T cell composition of the present invention is administered to the patient by intradermal or subcutaneous injection. In another embodiment, the T-cell composition of the invention is preferably administered by i.v. injection. The T-cell composition may be injected directly into a tumor, lymph node or location of infection.

In certain embodiments of the invention, cells activated and expanded using the methods described herein or other methods known in the art for expanding T cells to therapeutic doses are administered to a patient in combination with (e.g., before, at the same time or after) any number of relevant forms of therapy, said forms of therapy including, but not limited to, treatment with: said agents such as antiviral therapy, cidofovir and leukocyte interleukin-2, cytarabine (also known as ARA-C) or natalizumab treatment in patients with MS or efalizumab treatment in patients with psoriasis or other treatment in patients with PML. In further embodiments, the T cells of the present invention may be used in combination with: chemotherapy, radiation, immunosuppressive agents such as, cyclosporin, azathioprine, methotrexate, mycophenolate mofetil and FK506, antibodies or other immunotherapeutic agents. In further embodiments, the cellular compositions of the present invention are administered to a patient in combination with a bone marrow transplant, utilizing a chemotherapeutic agent such as fludarabine, external beam radiation therapy (XRT), cyclophosphamide (e.g., before, while or after). For example, in one embodiment, the subject may undergo standard treatment with high-dose chemotherapy followed by peripheral blood stem cell transplantation. In some embodiments, following transplantation, the subject receives an infusion of the expanded immune cells of the present invention. In an additional embodiment, the expanded cells are administered before or after a surgical procedure.

The dose of the above treatment administered to a patient will vary with the precise properties of the condition being treated and the recipient of the treatment. The ratio of doses administered by a person may be implemented according to accepted practice in the art. Typically, from 1 × 10⁹ to 1 × 10¹¹ double negative T cells of the present invention may be administered to a patient per treatment or per course of treatment, for example, by intravenous infusion.

### The main advantages of the present invention include:

1) Formulation and process improvement for efficient in vitro expansion method of human DNT cells suitable for clinical use.
2) Formulation and process improvements that eliminate the need for human AB serum and fetal bovine serum (FBS).
3) Improved formulation and process to culture freshly enriched DNT cells or recovered DNT cells after freezing.
4) Improved formulation and process allows for single collection/frozen storage and multiple recovery cultures for multiple transfusions or multiple patient use.
5) Improved formulation and process allows for cell expansion quantity can reach 1×10⁹ cells/mL whole blood and above.
6) Improved formulation and process to ensure ≥85% purity of DNT cells (CD3⁺CD4⁻CD8⁻) in the final product.
7) Formulation and process improvement to ensure efficient tumor killing activity (biological effectiveness) of ≥50% of cells in vitro.

The present invention is further described below in connection with specific embodiments. It should be understood that these embodiments are intended to illustrate the invention only and are not intended to limit the scope of the invention. Experimental methods for which specific conditions are not indicated in the following embodiments generally follow conventional conditions, such as those described in Sambrook et al, Molecular Cloning: A Laboratory Manual (New York: Cold Spring Harbor Laboratory Press, 1989), or as recommended by the manufacturer. Percentages and parts are percentages by weight and parts by weight unless otherwise stated.

### Example 1: In vitro expansion culture of magnetic bead-enriched DNT cells

### 1.1 Enrichment of healthy donor DNT cells by magnetic beads

Collect 20-200 ml of peripheral blood from healthy donors into heparin-containing sodium tubes. The white membrane layer cells were separated by lymphocyte isolation solution, and CD4 magnetic beads were added first to remove CD4⁺ cells, then CD8 magnetic beads were added to remove CD8⁺ cells, and the cells obtained were DNT cells with CD4⁺ and CD8⁺ removed.

### 1.2 In vitro expansion of cultured DNT cells

The T25 culture flasks were first coated with anti-human CD3 monoclonal antibody (10 µg/mL), DNT cells obtained in step 1.1 were adjusted to a concentration of 1×10⁶ to 4×10⁶ cells/mL in AIM-V medium (containing 10% donor plasma and 500 IU/mL of recombinant human interleukin 2) and placed in the previously coated T25 culture flasks. Incubate at 37°C in a 5% CO₂ incubator.

On day 3, DNT cells were adjusted to a concentration of 1×10⁶ to 1×10⁶ cells/mL with fresh AIM-V medium (containing 10% donor plasma and 500 IU/mL of recombinant human interleukin 2) and continued to be cultured.

On day 5, DNT cells were adjusted to a concentration of 1×10⁶ to 2×10⁶ cells/mL with fresh AIM-V medium (containing 10% donor plasma and 500 IU/mL of recombinant human interleukin 2) and continued to be cultured.

The phenotype and viability of DNT cells were examined on day 7, and DNT cells were adjusted to a concentration of 1×10⁶ to 3×10⁶ cells/mL with fresh AIM-V medium (containing 50 ng/mL of anti-human CD3 monoclonal antibody and 500 IU/mL of recombinant human interleukin 2) and continued to be cultured.

On day 9, DNT cells were adjusted to a concentration of 1×10⁶ to 3×10⁶ cells/mL with fresh AIM-V medium (containing 50 ng/mL of anti-human CD3 monoclonal antibody and 500 IU/mL of recombinant human interleukin 2) and continued to be cultured.

On day 10, DNT cells were examined for phenotype and viability, and DNT cells were adjusted to a concentration of 1×10⁶ to 3×10⁶ cells/mL with fresh AIM-V medium (containing 50 ng/mL of anti-human CD3 monoclonal antibody and 500 IU/mL of recombinant human interleukin 2) and transferred to culture bags for further culture.

On day 12, DNT cells were adjusted to a concentration of 1×10⁶ to 3×10⁶ cells/mL with fresh AIM-V medium (containing 50 ng/mL of anti-human CD3 monoclonal antibody and 500 IU/mL of recombinant human interleukin 2) and transferred to culture bags for further culture.

The phenotype and viability of DNT cells were measured on day 14, and the concentration of anti-human CD3 monoclonal antibody in AIM-V medium was reduced to 25 ng/mL to reduce impurity interference in the final product and to improve the purity of DNT cells. The DNT cells were adjusted to a concentration of 1×10⁶ to 3×10⁶ cells/mL with fresh AIM-V medium (containing 25 ng/mL of anti-human CD3 monoclonal antibody and 500 IU/mL of recombinant human interleukin 2) and continued to be cultured.

On days 15-16, as needed, DNT cells were adjusted to a concentration of 1 × 10⁶ to 3 × 10⁶ cells/mL with fresh AIM-V medium (containing 25 ng/mL of anti-human CD3 monoclonal antibody and 500 IU/mL of recombinant human interleukin 2) and continued to be cultured.

On day 17 to 18, DNT cells were harvested as needed.

### 1.3 Harvesting DNT cells to make DNT cell preparation

On day 17-18, harvest DNT cells as needed. The cells were collected in 250 ml pointed-bottom centrifuge bottles, centrifuged at 900 × g for 10 min, and then washed with solvent. The collected DNT cells were adjusted to a concentration of 0.5~1×10⁸ cells/mL with solvent, and this is the finished DNT cell preparation, which can be used for clinical use after passing quality control.

### Example 2: Magnetic bead-enriched DNT cells were lyophilized and recovered for in vitro expansion culture

### 2.1 Enrichment of healthy donor DNT cells with magnetic beads

Same as 1.1 above.

### 2.2 Obtained DNT cells for freezing and storage

DNT cells obtained in step 2.1 were washed once with 0.9% saline, resuspended in lyophilization solution and adjusted to a cell concentration of 3-5×10⁶ cells/mL, frozen in liquid nitrogen, and recovered for in vitro expansion culture as needed.

### 2.3 Recovery of frozen DNT cells

The frozen DNT cells from step 2.2 were removed from the liquid nitrogen and thawed in a 37°C incubator. The thawed DNT cells were washed once with AIM-V medium (containing 500 IU/mL of recombinant human interleukin 2) and resuspended with AIM-V medium (containing 10% donor plasma and 500 IU/mL of recombinant human interleukin 2).

### 2.4 In vitro expansion of cultured DNT cells

Same as 1.2 above.

Harvest DNT cells on day 17 to 18 as needed.

### 2.5 Harvest DNT cells to make DNT cell preparation

Same as 1.3 above.

### Example 3: In vitro expansion culture of sedimentation-enriched DNT cells

### 3.1 Healthy donor DNT cell enrichment

Collect 20 to 200 ml of peripheral blood from healthy donors into containing heparin tube. CD4⁺ and CD8⁺ T cells were removed using CD4/CD8 lymphocyte remover (RossettSep, StemCell), and PBMCs were isolated with an equal volume of Ficoll-Hypaque solution. The cells thus obtained were the DNT cells with CD4⁺ and CD8⁺ removed. The isolated cells were washed once with 0.9% saline and cultured for in vitro expansion.

### 3.2 In vitro expansion culture of DNT cells

Same as 1.2 above.

On day 17 to 18, harvest DNT cells as needed.

### 3.3 Harvest DNT cells to make DNT cell preparation

Same as 1.3 above.

### Example 4: Sedimentation-enriched DNT cells were lyophilized and recovered for in vitro expansion culture

### 4.1 Enrichment of healthy donor DNT cells

Same as 3.1 above.

### 4.2 Obtained DNT cells for freezing and storage

Same as 2.2 above.

### 4.3 Recovery of frozen DNT cells

Same as 2.3 above.

### 4.4 In vitro expansion of cultured DNT cells

Same as 1.2 above.

On day 17 to 18, harvest DNT cells as needed.

### 4.5 Harvest DNT cells to make DNT cell preparations

Same as 1.3 above.

### Example 5: Application Example and Effectiveness Data

In this Example, CD3⁺CD4⁻CD8⁻ T (Double Negative T, DNT) cells are used as an example to verify the process and effect of in vitro expansion by the above-mentioned efficient in vitro expansion method for universal clinical human DNT cells.

### Application Example 1

According to the method of Example 1, DNT cells obtained by removing CD4⁺ and CD8⁺ with magnetic beads were directly expanded and cultured in vitro using the method of the present invention (recombinant human interleukin 2 at a concentration of 500 IU/mL and a two-stage reduction in the amount of anti-human CD3 monoclonal antibody (50 ng/mL in the first stage and 25 ng/mL in the second stage), without the use of interleukin 4 and without the use of AB serum). Cells were collected on day 17-20, and cell activity, phenotype and total number of cell expansions were detected.

### Application Example 2

In accordance with the method of Example 2, DNT cells obtained by removing CD4⁺ and CD8⁺ with magnetic beads were lyophilized and then recovered and expanded and cultured in vitro using the method of the present invention (recombinant human interleukin 2 at a concentration of 500 IU/mL and a two-stage reduction of anti-human CD3 monoclonal antibody (50 ng/mL in the first stage and 25 ng/mL in the second stage), without interleukin 4 or AB serum). The cells were collected on day 17-20 and tested for cell activity, phenotype, and total number of cell expansions.

### Application Example 3

DNT cells obtained by removing CD4⁺ and CD8⁺ with CD4/CD8 lymphocyte remover according to the method of Example 3 were directly expansion cultured in vitro using the method of the present invention (recombinant human interleukin 2 at a concentration of 500 IU/mL, a two-stage reduction in the amount of anti-human CD3 monoclonal antibody (50 ng/mL in the first stage and 25 ng/mL in the second stage), without using interleukin 4 and without using AB serum)), and cells were collected on day 17-20 to detect cell activity, phenotype, and total number of cell expansions.

### Application Example 4

DNT cells obtained by removing CD4⁺ and CD8⁺ with CD4/CD8 lymphocyte remover according to the method of Example 4 were lyophilized and then recovered using the method of the present invention (recombinant human interleukin 2 at a concentration of 500 IU/mL, a two-stage reduction in the amount of anti-human CD3 monoclonal antibody (50 ng/mL in the first stage and 25 ng/mL in the second stage), without using interleukin 4 and without using AB serum) for in vitro expansion culture, and cells were collected on days 17-20 to detect cell activity, phenotype, and total number of cell expansions.

### Comparisons 1

In accordance with the prior art, recombinant human interleukin 2 (500 IU/mL), recombinant human interleukin 4 (2 ng/mL), recombinant human interleukin 7 (8 ng/mL), recombinant human interleukin 12 (5 ng/mL), 5% autologous plasma and 6% AB serum were added to the culture medium. The specific cell culture procedure was the same as the above application example.

### Comparisons 2

Referring to existing literature (Lee JB et al, Clin Cancer Res. 25(7):2241-2253, 2019), 250 IU/mL of interleukin 2 with 100 ng/mL of anti-human CD3 monoclonal antibody was used. The specific cell culture procedure was the same as described above for the application examples.

The cells from the aforementioned application examples and comparisons were sampled on day 1, day 7, day 14, and day 17 for total cell numbers, comparing the change in the total number of cells, the cell growth curves of the application example and the comparisons are shown in Figure 1, as seen in Figure 1, the cells of the application example show a significant increase in cell number from day 7, while the increase in cell number is not evident until day 14 for Comparisons 1, and a small increase in cell number from day 7 to day 14 for Comparisons 2, and a significant increase in cell number only begins on day 14. It shows that the method of the present invention can expand more DNT cells at the same culture time point compared to the comparisons.

DNT cells were collected on day 17, and the cell viability, phenotypic purity and DNT expansion folds are compared between the application example and the comparisons, and the results are listed in Table 1.

**Table 1 Cell viability, phenotypic purity and expansion folds of application examples and comparisons**

| **Groups** | **DNT cells viability** | **DNT cells Phenotypic purity** | **DNT cells expansion folds** |
|---|---|---|---|
| application example 1 | 93.0% | 96.20% | 15873.68 |
| application example 2 | 92.2% | 98.20% | 10778.95 |
| application example 3 | 90.0% | 93.85% | 20948.08 |
| application example 4 | 89.6% | 95.55% | 10819.41 |
| comparisons 1 | 91.8% | 92.00% | 8827.93 |
| comparisons 2 | 90.4% | 92.90% | 9402.65 |

In Table 1, Application Example 1 and Application Example 3 were cultured directly after freshly isolated DNT cells, as were Comparisons 1 and Comparisons 2. Comparing the DNT cell expansion folds, the DNT cell expansion folds obtained in vitro expansion by the method of the present invention using magnetic bead enrichment and sedimentation enrichment of DNT cells are 15873.68 and 20,948.08, respectively, which are much higher than the 8827.93 and 9402.65 folds of Comparisons 1 and Comparisons 2, respectively, while the differences in cell phenotype and cell viability are not significant. Thus, DNT cells enriched by two different methods using the expansion technique of the present invention result in a larger number of DNT cell products.

The isolated DNT cells were lyophilized, recovered and expanded in vitro by the method of the present invention to obtain Application Example 2 and Application Example 4 (Table 1). Compared with the direct culture without lyophilization of Comparisons 1 and Comparisons 2, two different methods were used to enrich DNT cells, and then the DNT cells expansion folds obtained from expanded in vitro after lyophilization of recovered DNT cells are 10,778.95-fold and 10,819.41-fold, respectively, which are comparable to the direct culture without lyophilization of Comparisons 1 (8827.93-fold) and Comparisons 2 (9402.65-fold), and the differences in DNT cell purity and cell viability are not significant.

### Application example 5

DNT cells enriched by removing CD4⁺ and CD8⁺ from peripheral blood were recovered after freezing in liquid nitrogen for 7, 14, and 30 days, and then expanded and cultured in vitro using the expansion method of the present invention (recombinant human interleukin 2 concentration of 500 IU/mL, two-stage reduction of anti-human CD3 monoclonal antibody dosage (50 ng/mL in the first stage and 25 ng/mL in the second stage), without using interleukin 4 or AB serum, and the cells were collected on day 17 and assayed for cell viability, purity, cell biological efficacy, and DNT cell expansion folds.

The cell viability, purity, cell biological efficacy, and DNT cell expansion folds of fresh whole blood-enriched DNT cells were compared with those of recovered DNT cells after freezing, and the results are listed in Table 2.

**Table 2 Comparative results of cell purity, biological efficacy and DNT cell expansion folds**

| **Groups** | **DNT cells viability** | **DNT cells Phenotypic purity** | **biological efficacy Percentage of tumor cells killed** | **DNT cells expansion folds** |
|---|---|---|---|---|
| Fresh whole blood enrichment | 90.23% ± 0.25% (n=3) | 96.48% ± 2.79% (n=3) | 76.87% ± 3.11% (n=3) | 6761 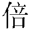 ∼21869 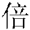 (n=3) |
| Recovered after 7 days of cryopreservation | 89.37% ± 1.46% (n=3) | 97.45% ± 1.66% (n=3) | 73.13% ± 5.95% (n=3) | 7745 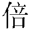 ∼11043 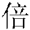 (n=3) |
| Recovered after 14 days of cryopreservation | 89.13% ± 1.17% (n=3) | 97.19% ± 2.66% (n=3) | 71.23% ± 12.95% (n=3) | 4709 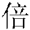 ∼10957 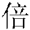 (n=3) |
| Recovered after 30 days of cryopreservation | 88.47% ± 5.19% (n=3) | 94.70% ± 1.37% (n=3) | 67.72% ± 14.91% (n=3) | 2961 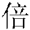 ∼8819 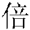 (n=3) |

As shown in Table 2, there are no significant differences in cell viability (>85%), cell purity (CD3⁺CD4⁻CD8⁻, >90%), tumor killing activity (>50) and in vitro expansion folds when compared to DNT cell batches cultured continuously for 17 days after fresh enrichment of DNT cells and DNT cells batches recovered and then cultured in vitro after freezing for different times (7 days, 14 days, 30 days), where the differences in expansion folds among batches may be related to the characteristics of DNT cells among different donors, but all of them can meet the clinical infusion requirements. Overall, the present invention can be applied to the in vitro mass expansion of DNT cells from freshly enriched DNT cells as well as recovered DNT cells after lyophilization. Not only the end-product DNT cell viability is above 85%, the purity is over 90%, the biological efficacy is over 50%, but also the DNT cell expansion folds can reach over 8,000 times.

The above embodiments confirm that the process provided by the present invention for freezing DNT cells at day0 followed by efficient in vitro expansion provides flexibility in cell preparation suitable for clinical treatment protocols, and further improves the production process based on the existing patented expansion technology to increase the in vitro expansion level of DNT cells to 8,000-fold order of magnitude in 17 days, enabling a single expansion of the resulting DNT cells to deliver a clinical therapeutic dose to multiple patients.

All publications mentioned herein are incorporated by reference as if each individual document was cited as a reference, as in the present application. It should also be understood that, after reading the above teachings of the present invention, those skilled in the art can make various changes or modifications, equivalents of which falls in the scope of claims as defined in the appended claims.

## Claims

1. An in vitro expansion method of a universal DNT cell, comprising the steps of:
(i) providing a starting sample I of peripheral blood obtained from a donor;
(ii) removing CD4⁺ and CD8⁺ T cells from the starting sample, thereby obtaining a sample II;
(iii) culturing the sample II in a culture system containing a medium suitable for the growth of a DNT cell, thereby obtaining a sample III; wherein the culture system comprises immobilized T-cell mitogen;
(iv) culturing the sample III in a culture system containing a medium suitable for DNT cell growth, thereby obtaining a sample IV; wherein the medium contains a soluble T-cell mitogen at a concentration of 30-80 ng/mL (preferably 40-60 ng/mL, more preferably 50 ng/mL);
(v) culturing the sample IV in a culture system containing a medium suitable for DNT cell growth, wherein the medium contains soluble T-cell mitogen at a concentration of 15-40 ng/mL (preferably 20-30 ng/mL, more preferably 25 ng/mL, more preferably soluble T-cell mitogen in an amount 1/2 of the added amount of the T-cell mitogen as described in sub-step (va), thereby obtaining a desired amount of universal DNT cell, as a sample V; and
(vi) collecting the sample V in a system containing a solution suitable for the preservation of the DNT cell; wherein the solution contains a human albumin, thereby obtaining the universal DNT cell for clinical use, as a DNT cell preparation;
wherein the culture systems of steps (iii) to (v) all contain 200-1000 IU/mL (preferably 300-700 IU/mL, more preferably 500 IU/mL) of recombinant human interleukin 2, and all do not contain recombinant human interleukin 4, and all do not contain AB serum.

2. The in vitro expansion method of claim 1, wherein the sample II is a solution of recovered cell after cryopreservation.

3. The in vitro expansion method of claim 1, wherein in the culture medium of the steps (iii) to (v), further comprising a cytokine selected from the group consisting of: IL-7, IL-12, IL-15, and a combination thereof.

4. The in vitro expansion method of claim 1, wherein the T cell mitogen is selected from the group consisting of: an antibody binding CD3, a lectin, a compound capable of stimulating the expansion of DNT cells, and a combination thereof.

5. The in vitro expansion method of claim 1, wherein in the step (iii), the initial concentration of DNT cells to be expanded in the culture system is from 1 × 10⁶ to 4 × 10⁶ cells/mL.

6. The in vitro expansion method of claim 1, wherein in step (iv), further comprising the detection of DNT cell phenotype and viability.

7. The in vitro expansion method of claim 1, wherein wherein in step (iv), further comprising the detection of DNT cell phenotype and viability.

8. The in vitro expansion method of claim 1, wherein in step (vi), comprising the steps of:
(via) collecting the DNT cells in the sample V by centrifugation.
(vib) washing the DNT cells with saline (or "solvent") containing 2.5% human albumin; and
(vic) adjusting the DNT cells to a concentration of 1 × 10⁸ cells/mL with saline (or "solvent ") containing 2.5% human albumin.

9. Use of an effective amount of the DNT cell obtained by the method of claim 1 for the preparation of a pharmaceutical composition or a preparation, the pharmaceutical composition or the preparation being used for:
(a) the prevention and/or treatment a tumor.
(b) the prevention and/or treatment of an infectious disease.
(c) the prevention and/or treatment of an autoimmune disease.
(d) the prevention and/or treatment of an allergic reaction.
(e) the prevention and/or treatment of a graft versus host disease; and/or
(f) the regulation of an immune response.

10. The use of claim 9, wherein the tumor is a tumor that is allogeneic to the DNT cell.
